# EUROPEAN PATENT SPECIFICATION

(11) **EP 3 072 483 B1**
(45) Date of publication and mention of the grant of the patent: **31.01.2018**
(21) Application number: 15161481.5
(22) Date of filing: 27.03.2015
(51) Int. Cl.: A61F 13/00, A61F 13/02

(54) **WOUND DRESSING**
WUNDAUFLAGE
PANSEMENT DE PLAIE

(43) Date of publication of application: 28.09.2016
(73) Proprietor: Absorbest AB, 590 39 Kisa (SE)
(72) Inventor: Rovaniemi, Rolf, 590 46 Rimforsa (SE)
(74) Representative: WSL Patentanwälte Partnerschaft mbB

(56) References cited:
- WO-A1-2014/204803
- US-A1- 2004 241 215
- US-A1- 2008 009 812
- US-A1- 2013 123 678
- US-A1- 2015 088 085

## Description

The present invention relates to a wound dressing having a top surface and a bottom surface with an absorbent core having a top surface and a bottom surface and comprising a superabsorbent substance and a cover, wherein the absorbent core is enclosed by the cover.

Wound dressings for covering weeping or exudating wounds exist in various types of designs. When applied to a wound the dressings cover the wound in order to prevent any further harm to it and in order to protect the wound from any exterior material which could penetrate the wound and lead to inflammation or infection. The healing process of the wound under the wound dressing may therefore be improved.

Exudate plays an essential role in healing of moist wounds because problems can occur when a wound produces too much exudate. For example, skin damage in the margin surrounding the wound referred to as periwound skin damage, an increased risk of critical contamination or infection of the wound or a delayed healing process may occur. Thus, for heavily exudating wounds the wound dressing is often equipped with an absorbent core comprising a superabsorbent substance. This superabsorbent substance has the capability of absorbing a large amount of liquid until being saturated.

The exudate from the wound contains biochemical compounds which support the wound healing. However, in case of an excessive amount of exudate in the wound or in the periwound region, the absorbent core experiences a faster saturation since it has to retain the exudate released by the wound with a high rate. A higher amount of bacteria could be accumulated in the exudate containing absorbent core since the exudate additionally serves as a growth medium for bacteria. The wound dressing should therefore be replaced regularly depending on the rate of exudate being absorbed by the absorbent core of the wound dressing.

A regular change of the wound dressing due to the saturation of the absorbent core requires both a facilitated removal of the wound dressing causing only a scarce pain to the patient and an easy application of a new wound dressing onto the wound.

Apart from the characteristics of absorption and retention of exudate the most important characteristics of wound dressings for strongly exudating wounds are a low adhesion to the wound surface as well as an easy removal from the wound and, moreover, a simple and reliable application onto the wound.

In the prior art, fixation of the wound dressing onto a patient's skin is mostly implemented by a self-adhesive or pressure sensitive glue covering the bottom surface of the wound dressing which attaches the wound dressing to the wound for a permanent fixation comparable to a sticking plaster. This attachment occurs via a slight pressure exerted on the wound dressing in direction to the patient's skin such that the pressure sensitive glue is brought in direct contact with the patient's skin and adheres to the patient's skin due to the physical interaction between the pressure sensitive glue and the patient's skin. Hereby it is important that the pressure sensitive glue adapts well to the skin and does not irritate the skin. Furthermore, the pressure sensitive glue should adhere well to the skin without causing damage to the skin when the wound dressing is removed. However, the pressure sensitive glue exhibits strong adhesive properties which involves two major disadvantages. If the wound dressing has been misplaced, a repositioning of the wound dressing is difficult and accompanied by a severe pain induced on a patient when removing the wound dressing. A further disadvantage is the severe pain in general, occurring when the wound dressing is removed, independent of a preceded misplacement.

In a further prior art, fixation of the wound dressing onto a patient's skin is achieved by using a non-adhesive wound dressing in combination with a bandage or an adhesive tape. After the wound dressing has been placed above the wound it is fixed by a bandage or an adhesive tape. However, the major disadvantage of this type of fixation is its complicated application procedure. The wound dressing can easily slip away or even fall down before the bandage or adhesive tape is applied.

US 2015/088085 A1 relates to a wound dressing including an absorbent core, a facing layer and a backing layer. The absorbent core is located between the facing layer and the backing layer. The wound dressing further includes an adhesive configured to attach the wound dressing to a patient's skin wherein the facing layer faces the skin.

It is therefore an object of the present invention to provide a wound dressing with an absorbent core which enables an easy repositioning of the wound dressing if it has been misplaced. It is further an object of the present invention to provide a wound dressing with an absorbent core which leads to a substantial decrease of pain caused for the patient when the wound dressing is removed. Another object of the present invention is to provide a wound dressing with an absorbent core which facilitates the application of the wound dressing onto the wound and reduces the possibility that the wound dressing slips away before being permanently fixed on the wound.

At least one of these objects is solved by a wound dressing with an absorbent core, having a top surface and a bottom surface, and comprising a superabsorbent substance and a cover, wherein the absorbent core is enclosed by the cover, wherein the wound dressing has an application aid in form of a pressure sensitive glue, wherein the pressure sensitive glue extends on an outer surface of the cover facing towards a wound, when the wound dressing is in use, and wherein the total surface covered by the pressure sensitive glue is smaller than the bottom surface of the absorbent core, wherein a 180° peel adhesion of the pressure sensitive glue is smaller than or equal to 0.68 N/mm.

In contrast to a wound dressing with a common permanent fixation on the wound, the application aid herein is defined by its characteristics of a non-permanent fixation enabling an easy repositioning and a regular replacement of the wound dressing. In order to fulfill this characteristics the application aid consists of a pressure sensitive glue attaching the wound dressing to the patient's skin, which has the essential property of a low adhesion when applied to the skin such that it peels off easily reducing pain induced on the patient.

In addition to a substantial decrease in pain for the patient, the regular change of the wound dressing made possible by the application aid has the consequence that a saturation of the absorbent core of the wound dressing, which is accompanied by a leakage of the absorbed wound fluid at the side portions of the wound dressing as well as by the risk of the accumulation of bacteria in the absorbent core, is prevented. An improved healing of the wound may therefore be realized.

With a wound dressing according to the present invention fixation of the wound dressing is facilitated. Due to the pressure sensitive glue the wound dressing can reliably be brought onto the skin without getting out of place. However, if the wound dressing is misplaced, it can also easily be peeled off the skin due to its low adhesion leading to a substantial relief of pain and can be repositioned.

In order to prevent that the exudate flow from the wound into the absorbent core is limited by the pressure sensitive glue, the total surface, on which the pressure sensitive glue extends, is smaller than the bottom surface of the absorbent core.

In one embodiment the pressure sensitive glue of the wound dressing has a 180° peel adhesion which is smaller than the 180° peel adhesion of a zinc oxide rubber resin glue which is commonly used for self-adhesive wound dressings with a permanent fixation. The 180° peel adhesion of the pressure sensitive glue given and discussed in this application is determined under the framework of the pressure sensitive tape council (PSTC) 101 test. A description of the test method is available online on the PSTC webpage, http://www.pstc.org/files/public/101.pdf, issued in October 2000 and last revised in May 2007.

In general, peel adhesion is defined as a measure of the strength of an adhesive bond between a tape and a test surface. Thus, the peel adhesion describes the force required to remove a pressure sensitive tape from a test surface at a controlled angle and at a standard rate. The dimension of the peel adhesion is given in force per width of the tape, resulting in the unit of measurement of Newton (N) per millimeter (mm).

In case of the 180° peel adhesion used for all values given in this application the test gives a measure of the adherence when peeled at 180° degree off a polished stainless steel test panel as test surface at a standard rate of 5 mm per second. The values of adhesion given herein were measured twenty minutes after the application of the pressure sensitive glue onto a test panel.

In an embodiment, 180° peel adhesion of the pressure sensitive glue is smaller than or equal to 0.2 N/mm, preferably smaller than or equal to 0.06 N/mm and most preferably smaller than or equal to 0.04 N/mm. However, the 180° peel adhesion of the pressure sensitive glue exceeds 0.004 N/mm in order to provide at least a minimal adhesive contact between the wound dressing and the patient's skin. Consequently, the wound dressing with its application aid provides a low adhesion onto the skin such that the removal and repositioning of the wound dressing does not cause a severe pain for the patient.

A quick adhesion of the wound dressing onto the patient's skin facilitates the fixation of the wound dressing onto the skin since the attachment occurs very fast. For example, a one-handed application of the wound dressing onto the wound can be realized very easily. Furthermore, the fast attachment to the skin minimizes the possibility that the wound dressing slips on the skin.

In an embodiment, the pressure sensitive glue of the wound dressing adheres quickly to the skin. A quick adhesion refers to a distance of 8 cm or less, which a rolling ball on the pressure sensitive glue covers during the rolling ball tack test until being stopped due to adhesion, preferably a distance of 3 cm or less, preferably a distance of 2 cm or less, and most preferably a distance of 1 cm or less.

One method to quantify the ability of an adhesive to adhere quickly to another surface is the so-called rolling ball tack test, one of the earliest of tack methods classified as PSTC 6. The description of the test method is published on the PSTC webpage, http://www.pstc.org/files/public/6.pdf, issued in October 1964 and last revised in October 2003. As quoted in this document, the rolling ball tack test constitutes "one measure of the capacity of the adhesive to form a bond with the surface of another material upon brief contact under virtually no pressure". Hereby, the sample to be tested is arranged on a perfectly horizontal working surface, which is a glass plate in case of the PSCT 6 test on which the values given in this application refer to, with its adhesive side up after having cleaned the raceway thoroughly. The raceway of an incline with the standard conditions of a height of 65 mm, inclined at 21.5° to the horizontal, as mentioned in the NPL Report No CMMT(A)176, May 1999, is aligned with the sample tape under test so that at least 30 cm of tape is exposed. A stainless steel ball with a diameter of 11.1 mm is then placed on the upper side of the release pin of the incline. When the ball is released it runs down the incline and rolls further on the adhesive tape until it is stopped due to adhesion. The distance from the point where the ball initially contacts the adhesive to where the ball stops is measured and provides a quantity of the adhesive tape's ability to adhere quickly to the specific test surface. All values given in this application referring to the rolling ball tack test are measured according to this described procedure.

An often used method for determining the tack of an adhesive is the so-called loop tack test referred to as FINAT test method (FTM) 9, but also known as PSTC 16. This test method belongs to the internationally approved standard methods which were developed by FINAT (an abbreviation of the French title: **F**ederation **IN**ternationale des fabricants et transformateurs d'**A**dhesifs et **T**hermocollants sur papiers et autres supports) for carrying out tests of materials for self-adhesive labels. A description of this test method apart from other test methods is available online (http://www.adhesivetest.com/resources/docs/FinatTestMethods.pdf). The loop tack test provides a means of measuring the tack of an adhesive, i.e. the method allows to compare the initial tack of different adhesive materials. The loop tack value of an adhesive coated layer is described by the force required to separate a loop of the adhesive tape with its adhesive coated layer outwards from a specified area of a glass plate with a width and length of 25 mm, to which the loop has been brought in contact, at a specific speed. Directly after its attachment to the glass plate the formed loop is pulled away from it with a speed of 5 mm per second. The maximum force necessary to completely separate each loop from the glass plate is recorded in Newton and gives a measure of the initial tack. As documented in the nolax data sheet describing the application of the Loop-Tack ("Quick Stick") - FINAT No. 9, 0101e - Revision 01, the adhesive tape hereby has a width of 25 mm and a length of 150 mm in the feed direction. These measures provide that it can be formed into a loop with a length of at least 10 mm marking the distance between the glass plate and the pulling machine when the loop is longitudinally extended. All values given in this application referring to the loop tack test are measured according to this described procedure.

In an embodiment, the tack of the pressure sensitive glue of the wound dressing determined in the framework of the FINAT No. 9, 0101e - Revision 01 test is smaller than or equal to 25 N, preferably smaller than or equal to 8 N, preferably smaller than or equal to 6 N and most preferably smaller than or equal to 5 N. These values provide an upper tolerance limit for the tack of the pressure sensitive glue used for the application aid of the wound dressing. Thus, the wound and the periwound skin are protected from a too high tack of the adhesive leading to a reduction of pain.

An example of such a pressure sensitive glue having the adhesive properties as indicated in this application is the nolax M11.1289, a pressure sensitive hot melt adhesive non-irritating to the skin with a very low peel value, i.e. a 180° peel adhesion of only 0.02 N/mm. Further specifications can be found in the corresponding technical data sheet, published on the nolax website http://nolax.com/technische-datenblaetter/e/e_nolax_m11.1289.pdf. All nolax adhesives are commercially available from nolax AG, Eichenstrasse 12, CH-6203 Sempach Station, Switzerland.

Another example of a pressure sensitive glue according to the present invention with a higher adhesion than the glue of the previous example is the nolax M11.180, a thermoplastic rubber having a 180°peel adhesion of 0.64 N/mm and a loop tack value of 24 N. Furthermore, the rolling ball tack test yields a distance of 6 cm covered by a stain-less steel ball rolling along the nolax M11.1289 glue until the ball is stopped due to its adhesion to the nolax M11.1289 glue. These given values regarding the adhesion characteristics can be found in the corresponding technical data sheet together with further properties of this specific glue.

In an embodiment, the pressure sensitive glue used for the application aid of the wound dressing is a hot melt adhesive, preferably a hot melt adhesive based on a thermoplastic polymer, or a cold melt.

A hot melt is a form of thermoplastic adhesive that is tacky when it is hot and solidifies in a few seconds to one minute producing a solid bond when the temperature decreases to room temperature. The hot melt is applied to the wound dressing when it is hot and provides a strong adhesion to the wound dressing due to its initially high tack. Its fast cool down under exposure to ambient air conditions leads to a strong reduction of the initial tack of a hot melt, finally resulting in a low adhesion to other surfaces. A hot melt can be based on various types of ingredients, for example, polymers, resins, stabilizers or waxes. Depending on their field of application the ingredients of the hot melt are chosen according to the requested properties of their usage regarding the adhesion on a substrate, a thermal behavior, the chemical stability and the hardness.

In contrast to hot melts, cold melts build bonds already at room temperature. They are usually based on bitumen as a main ingredient.

In an embodiment, the pressure sensitive glue used for the application aid of the wound dressing is a hot melt which comprises at least one of the following ingredients: a thermoplastic elastomer, an acryl, an ethylene-acrylate-elastomer, a polyolefin (PO), a low density polyethylene (LDPE) or a high density polyethylene (HDPE), a polybutene-1, a polyamide (PA), a polyester-elastomer (TPE-E), a polyurethane-elastomer (TPE-U), a styrene-butadiene-styrene (SBS), a styrene-isoprene-styrene (SIS), a styrene-ethylene/butylene-styrene (SEBS) or a styrene-ethylene/propylene (SEP) or a combination thereof.

In an embodiment, the pressure sensitive glue facing towards the skin during the usage of the wound dressing is shielded by a protective film before its application onto the skin. Preferably, the protective film is a non-adhesive silicon paper as it is already used in a wide range of applications for self-adhesive glues. The protective film preserves the durability of the pressure sensitive glue and keeps exterior particles from the ambient air away. Before applying the wound dressing onto the skin the protective film has to be removed such that the pressure sensitive glue can be brought into contact with the patient's skin.

There is a large variety of implementations how the pressure sensitive glue may cover the bottom surface of the wound dressing being in contact to the skin when the wound dressing is applied. A complete coverage of the bottom surface with the pressure sensitive glue has two major disadvantages. Firstly, the surface covered by the pressure sensitive glue is less permeable for the exudate from the wound, thus, the rate of absorption of exudate is reduced. Secondly, a larger surface covered by the pressure sensitive glue signifies that a larger surface of the patient's skin comes into contact with an adhesive material and can therefore be harmed to a certain extent although the pressure sensitive glue provides low adhesion properties. It can therefore be regarded as more advantageous if the pressure sensitive glue does not cover the whole bottom surface of the wound dressing, but covers only a minor part. However, the pressure sensitive glue should be applied onto the bottom surface of the wound dressing such that it can guarantee a loose fixation onto the wound without slipping away easily.

In an embodiment, the pressure sensitive glue used for the application aid of the wound dressing is arranged in form of at least two stripes which extend along the long edges or the short edges of the outer bottom surface of the cover. Such an arrangement enables a reliable fixation of the wound dressing onto the patient's skin neither blocking the exudate transport into the absorbent core nor producing a higher pain level than necessary in case of the removal of the wound dressing.

In an embodiment, the total surface of the outer surface of the cover, which is covered by the pressure sensitive glue, is smaller than 30%, preferably smaller than 20%, compared to the area of the bottom surface of the absorbent core. As mentioned above, the limitation of the ratio between the surface covered by the pressure sensitive glue and the bottom surface of the absorbent core is useful for improving both the transport of exudate from the wound into the absorbent core and a further prevention of the patient's pain.

In an embodiment, less than 40%, preferably less than 20%, of the outer surface of the cover covered by the pressure sensitive glue overlaps with, i.e. covers, the bottom surface of the absorbent core such that the blocking of the transport of exudate from the wound to the absorbent core by the pressure sensitive glue is limited. Thus, this arrangement increases additionally the absorption rate of exudate from the wound dressing by reducing the covering of the bottom surface of the absorbent core by the pressure sensitive glue.

In an embodiment, the pressure sensitive glue of the wound dressing is arranged in form of two adhesive stripes being parallel to each other and extending along opposite edges of the exterior bottom surface of the cover.

Wound dressings are in general intended as disposable items for reasons of hygiene. The wound dressing comprises a layered structure comprising at least a cover and an absorbent core which is enclosed by the cover. A wide range of suitable structures and materials may be used for the wound dressing.

An absorbent core having a superabsorbent substance, which in the following text may also be denoted as a superabsorbent core, extracts and stores liquid exudates from a wound, to which the wound dressing is applied to.

The absorbent core may be any structure suitable to absorb exudate from the wound and comprising a superabsorbent substance. The material of the absorbent core may comprise any one of a group consisting of cellulose, regenerated cellulose as viscose, in particular cellulose fluff or regenerated cellulose fluff, air-laid cellulose or air-laid generated cellulose, tissue paper, a non-woven, a textile fabric, a foam, an alginate, ALT, and a hydrocolloid or a combination thereof. In one embodiment, the absorbent core comprises a spun laced web material of 100% pure cellulose or a 100% regenerated cellulose as viscose. In another embodiment, the absorbent core comprises a mixture of pure cellulose or regenerated cellulose and synthetic fibres. In yet another embodiment, the absorbent core comprises a non-woven tamponade or a pad containing sodium carboxyl methyl cellulose and regenerated cellulose, as it is commercially available under the trade name aquacel*®* from ConvaTec (Germany) GmbH of Munich, Germany.

Superabsorbent substances in the sense of the present application are materials being able to absorb and retain large volumes of water in aqueous solutions. Superabsorbent substances falling into this category are for example modified starch, polymerized polyvinyl alcohol (PVA) and polyethylene oxide (PEO) which are all hydrophilic and have a high affinity to water. When chemically or physically cross-linked, these polymers are water-swellable but not water-soluble. The aforementioned superabsorbent substances have been known for a long time.

In a particular embodiment of the present invention, the superabsorbent substance is a superabsorbent polymer (SAP), in particular in form of (granular) particles or fibres. In an embodiment, such a SAP is made from polymerization of acrylic acids blended with sodium hydroxide in the presence of an initiated form poly-acrylic acid sodium salt (sometimes referred to a sodium polyacrylate).

When the absorbent core has already absorbed a large amount of exudate from the wound it is swollen. In order to prevent that either the patient's wound and periwound skin or the patient's clothing come into direct contact with the swollen absorbent core, the wound dressing in an embodiment further comprises a layer or a multilayer as a backing layer or a facing layer. The facing layer and the backing layer together form the cover, which prevents a direct contact between the absorbent core and the wound surface or the patient's clothing, respectively.

In an embodiment, the material of the facing layer consists of a non-woven which may either be a spun bond non-woven or a spun bond-melt blown-spun bond (SMS) non-woven, or a three-dimensional film of polyethylene, polyamide, polyester or a laminate made of those materials.

A non-woven fabric in the sense of the present application is a material made of at least one layer of fibres that have been formed to a web and consolidated in a next step. In particular, consolidation of the non-woven fabric may be achieved by friction and/or cohesion and/or adhesion, for example by needling, felting, spun-lacing, melting or heat embossing.

In an embodiment, the facing layer has a double layer structure wherein the two layers differ in their ingredients. The layer adjoining the absorbent core is build up from a 10 to 30 gsm (g/m²) non-woven, preferably 15 to 19 gsm (g/m²), against the absorbent core. The second layer is an outer layer which covers the layer adjoining the absorbent core and is a film.

Whenever in this context of the present application a weight is given in grams per square meter (g/m²) it means that a square meter of the respective material weighs to the amount of grams given. This weight in the paper industry is commonly denoted as paper weight, wherein the unit grams per square meter is identical to gsm. It is commonly named an area weight. When within this application a weight of superabsorbent polymers is given in grams per square meter (g/m²), it denotes the area weight of the superabsorbent polymers when taken on their own without fibres. Whenever a weight is given in the context of this application, it is given for the material in its unwetted state.

In an embodiment, the cover of the wound dressing entirely encloses the absorbent core such that the cover is sealed by a hot melt. Either one seam extends or two seams extend along one or both of the lateral surfaces being parallel to each other and perpendicular to the plane of the upper and lower surface of the absorbent core.

In an embodiment, the cover of the wound dressing comprises a facing layer in form of either a single-layer or a multi-layer facing the wound and covering at least partly the bottom surface of the absorbent core and a backing layer in form of either a single-layer or a multi-layer covering at least partly the top surface of the absorbent core.

By providing the facing layer and the backing layer as distinct and separate layers, their properties and functionalities can be individually designed as needed.

In an embodiment the facing layer is made of a material selected of a group comprising a non-woven fabric, a perforated film and a foam based on polyurethane, polyurethane or silicon or a combination thereof.

In a further embodiment the facing layer comprises a non-woven fabric consisting of synthetic or cellulose fibres, wherein the fibres of the non-woven fabric are orientated such that they predominantly extend in a direction perpendicular to the extension of the facing layer. Such orientation of the fibres in the non-woven fabric is achieved by orienting the fibres during the fabrication process, in particular during spun lacing or needling.

It is further useful if in an embodiment the facing layer comprises a hydrophobic or hydrophilic and/or bactericidal or bacteriostatic agents.

In an embodiment of the present invention the backing layer serves as a clothing protection. The backing layer is thus advantageously made of a breathable non-woven fabric or a perforated film. The backing layer further is hydrophobic and prevents that exudate stored in the absorbent core can leave the wound dressing towards its top surface by penetrating through the backing layer and thereby contaminating a patient's clothing.

The backing layer may form the outermost layer of the wound dressing at its top surface facing away from the wound, which if applicable is brought into contact with the a patient's clothing. However, there may be embodiments, wherein there are further layers on top of the backing layer, i.e. between the backing layer and a patient's clothing, providing additional functionalities for the wound dressing as a border for permanent fixation on the skin.

In an embodiment of the present invention, the material of the backing layer is selected of a group comprising a spun bond non-woven, a spun bond-melt blown-spun bond (SMS) non-woven, a spun bond-melt blown-spun bond-melt blown-spun bond (SMSMS) non-woven, a laminate of a non-woven material and a breathable film like a breathable textile back sheet (BTBS), and a breathable film, e.g. a polyurethane film.

In an embodiment the backing layer and the facing layer are joined together by a seam located such that it surrounds the absorbent core. This design is called a sandwich design, wherein the backing layer and the facing layer are located above and below the absorbent core resembling two slices of toast. In this sandwich design the respective surfaces of the facing layer and the backing layer which are in touch with the absorbent core are also glued together.

In another embodiment, the cover of the wound dressing comprises a facing layer and a backing layer. When the wound dressing is in use, the facing layer faces the wound and covers the bottom surface of the absorbent core entirely while the facing layer is folded towards the top surface of the absorbent core, thereby forming two folded sections. The backing layer is attached to these folded sections of the facing layer by a seam. Considering this formed tubular structure, the cross-section of the folded facing layer represents a C-shaped structure. The application aid of the wound dressing is located on a section of the facing layer. This design is denoted a tubular design.

In an embodiment of the tubular design those edges of the facing layer, which during the manufacturing process run in transport direction of the different materials assembled to form the dressing are folded towards the top surface of the absorbent core. In the next step those seams which run perpendicular to the transport direction are formed between the respective surfaces of the facing layer and the backing layer which are in touch with the absorbent core.

However, in an alternative embodiment of the tubular design all four edges of the facing layer are folded towards the top surface of the absorbent core and the backing layer is attached to these folded sections by a seam.

In another embodiment, the above-mentioned backing layer covers the top surface of the absorbent core entirely and is folded towards the bottom surface of the absorbent core forming two folded sections. The facing layer is attached to the folded sections of the backing layer by a seam. Considering this formed tubular structure, the cross-section of the backing layer represents a C-shaped structure. Such a structure has the advantage that a side leakage, i.e. a leakage of the absorbed wound fluid at the side portions of the wound dressing, is effectively eliminated since the backing layer, especially a BTBS backing layer, completely encloses the lateral edges of the wound dressing and, thus, serves as a barrier for exudate due to its impermeable characteristics. In this embodiment the application aid is located on a section of the facing layer and may also cover a part of the folded sections of the backing layer covering the bottom surface of the absorbent core.

In yet another embodiment, a sterile kit is provided which comprises a wound dressing according to one of the embodiments of the present invention as described above and a permanent fixation.

The permanent fixation comprising a covering layer and a pressure sensitive glue has a larger dimension than the top surface of the wound dressing. The pressure sensitive glue providing the adhesive contact between the covering layer and the skin has a stronger adhesion to the skin than the pressure sensitive glue of the application aid. For example, the 180° peel adhesion of the pressure sensitive glue of the permanent fixation is higher than the 180° peel adhesion of the pressure sensitive glue of the application aid. Thus, the pressure sensitive glue of the permanent fixation is a strongly self-adhesive glue.

In one embodiment, the permanent fixation of a sterile kit covers the top surface of the wound dressing and extends at least 20 mm beyond the edges of the top surface of the wound dressing.

One explicit example of a pressure sensitive glue covering the bottom surface of the permanent fixation is the nolax M11.228, a pressure sensitive hot melt adhesive non-irritating to the skin which bases on thermally rearranged (TR) polymers. According to the corresponding technical data sheet, which is published on the nolax webpage http://nolax.com/technische-datenblaetter/e/e_nolax_m11.228.pdf, the nolax M11.228 has a 180° peel adhesion of 1.04 N/mm. Thus, it exceeds by far the respective value obtained when measuring the 180° peel adhesion of a pressure sensitive glue which is used for the application aid. Due to its strong adhesion it is well suited for a permanent fixation onto the skin. According to the FTM 9, providing a means of measuring the tack of an adhesive, the loop tack value results in a maximum force of 38 N which is necessary in order to separate a loop of the adhesive tape material from a polished glass plate, to which the loop has been brought in contact with before. This high tack value of the pressure sensitive glue, exceeding by far the tack value of a pressure sensitive glue used for the application aid guarantees a reliable permanent fixation on the skin and prevents a later slipping of the wound dressing on the skin. Furthermore, the rolling ball tack test, used for quantifying the ability of an adhesive to adhere quickly to another surface, yields a distance of larger than 80 cm covered by a stainless steel ball rolling along the nolax M11.228 glue until the ball is stopped due to its adhesion to this specific nolax pressure sensitive hot melt.

However, the nolax M11.228 only represents one explicit example of a pressure sensitive glue used for the permanent fixation in terms of the present invention. Other types of pressure sensitive glues may also be used for the permanent fixation as long as they meet the necessary requirements, e.g. a high adhesion, according to the present invention.

In an embodiment, the permanent fixation comprises a covering layer which is a plastic film having a high elasticity and a good moisture vapor transmission rate (MVTR), but is hydrophobic such that it does not let water from the outside pass through the film, as it may occur when washing or taking a shower.

In another embodiment, the permanent fixation is made of a transparent polyethylene film coated with a polyacrylate adhesive non-irritating to the skin. The thin film is impermeable to germs as well as impermeable to moisture allowing the patients to wash or to take a shower. The stretchable film having a high elasticity conforms smoothly to body contours and adapts well to movements.

Further advantages, features and applications of the present invention will become apparent from the following description of embodiments and the corresponding figures attached.
Figure 1 shows a schematic top view onto the bottom surface of a wound dressing facing towards the patient's skin according to an embodiment of the present invention.
Figure 2 shows a schematic top view onto the bottom surface of a wound dressing facing towards the patient's skin according to an embodiment of the present invention.
Figure 3 displays a schematic top view onto the bottom surface of a wound dressing facing towards the patient's skin according to an embodiment of the present invention.
Figure 4 shows a schematic top view onto the bottom surface of a wound dressing with an elliptical shape facing towards the patient's skin according to an embodiment of the present invention.
Figure 5 shows a schematic top view onto the bottom surface of a wound dressing with an elliptical shape facing towards the patient's skin according to an embodiment of the present invention.
Figure 6 demonstrates a schematic top view onto the bottom surface of a wound dressing with an elliptical shape facing towards the patient's skin according to an embodiment of the present invention.
Figures 7A and 7B display alternative schematic cross-sectional views of wound dressings along line A-A of figure 1 with different designs of the cover formed by the backing and facing layers according to two embodiments of the present invention.
Figures 8A and 8B show alternative schematic cross-sectional views of wound dressings along line B-B in figures 1 and 3, respectively, with different seals on a wound dressing's short edges according to two embodiments of the present invention.
Figure 9 displays a schematic view onto the bottom surface of a wound dressing kit according to an embodiment of the present invention.
Figure 10 shows a schematic cross-sectional view of a wound dressing in combination with a permanent fixation according to an embodiment of the present invention.

In the following description of the figures, elements having equivalent functionality are denoted by identical reference numbers. The figures only represent schematic views and are not true to scale.

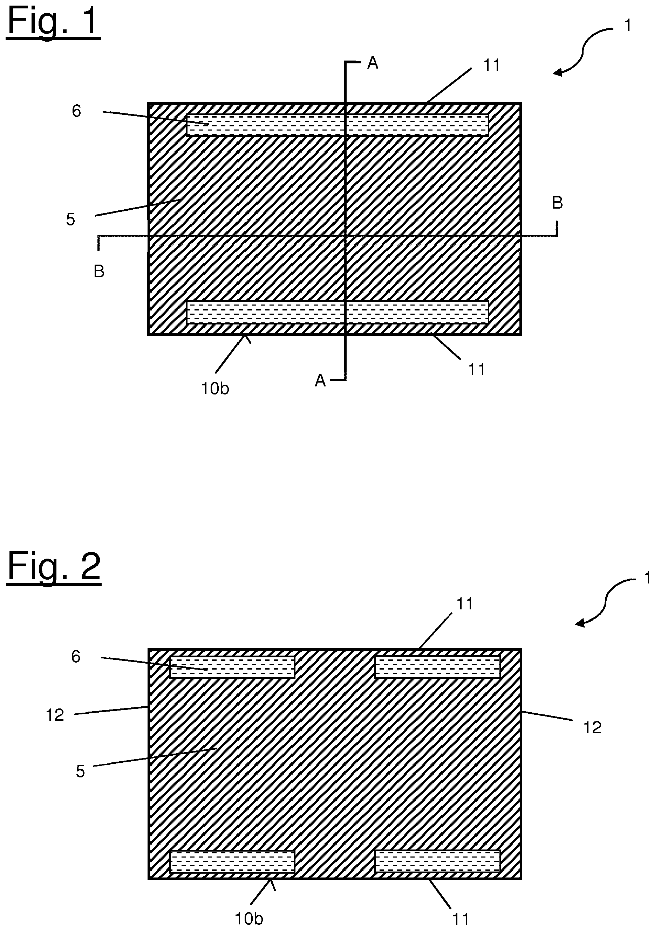

Figure 1 shows a schematic view onto the bottom surface 10b of a wound dressing 1 according to the present invention. The bottom surface 10b of the wound dressing 1 of figure 1 is in contact with the wound in case of its application. The bottom surface 10b is formed by a facing layer 5 facing the wound, on which an application aid 6 is applied. In the figures the facing layer 5 is depicted as a transparent layer in order to enhance the understanding of the figures. The bottom surface of the absorbent core being in direct contact with the facing layer 5 lies in a deeper plane with respect to the bottom surface 10b of the wound dressing 1 and is therefore not labelled in figure 1 but visible as hatchings due to the transparently pictured facing layer 5.

The absorbent core comprises a superabsorbent substance which is a superabsorbent polymer (SAP) in form of granular particles. In this embodiment, such a SAP is made from polymerization of acrylic acids blended with sodium hydroxide in the presence of an initiated form poly-acrylic acid sodium salt (sometimes referred to a sodium polyacrylate).

The facing layer 5 in this particular embodiment shown in figure 1 is made of a white hydrophilic non-woven fabric consisting of polypropylene fibres in order to provide a good transport of exudate from a wound to the absorbent core.

In the embodiment of figure 1, the application aid 6 forms two stripes of pressure sensitive glue being parallel to each other and extending along the long sides of the opposite edges 11 of the bottom surface 10b of the wound dressing 1. Before the application of the wound dressing 1 onto the wound the pressure sensitive glue is covered by a removable protective film which, however, is not shown in figure 1. These two stripes of pressure sensitive glue being parallel to each other are arranged such that the total surface of the outer surface of the cover covered by the pressure sensitive glue is smaller than 20 per cent of the bottom surface of the absorbent core. This requirement provides that the exudate transport from the wound to the absorbent core 2 is blocked as little as possible by the pressure sensitive glue.

The pressure sensitive glue used for the application aid 6, as it is applied in the embodiment shown in figure 1, is a nolax M11.1289 glue, a pressure sensitive hot melt adhesive non-irritating to the skin with a very low peel value. As specified in the corresponding technical data sheet, published on the nolax website http://nolax.com/technische-datenblaetter/e/e_nolax_m11.1289.pdf, the nolax M11.1289 has the following adhesive properties. The 180° peel adhesion of the nolax M11.1289 according to the PSTC 101 test method, measured 20 minutes after its attachment on a polished stainless steel plate, results in 0.02 N/mm. The rolling ball tack test according to the PSTC 6 test method, used for quantifying the ability of an adhesive to adhere quickly to another surface, yields a distance of 1 cm covered by a stainless steel ball rolling along the nolax M11.1289 glue until the ball is stopped due to its adhesion to this specific nolax pressure sensitive hot melt. According to the FTM 9, providing a means of measuring the tack of an adhesive, the loop tack value results in a maximum force of 3.3 N which is necessary in order to separate a loop of the adhesive tape material with its adhesive coated layer outwards from a polished glass plate, to which the loop has been brought in contact with, wherein the adhesive tape material and the glass plate both have a width of 25 mm.

However, the nolax M11.1289 only represents one explicit example of a pressure sensitive glue used for the application aid 6 in terms of the present invention. Other types of pressure sensitive glues may also be used for the application aid 6 as long as they meet the necessary requirements for an application aid 6, e.g. a very low adhesion, corresponding to the present invention.

Figure 2 shows a schematic view onto the bottom surface 10b of a wound dressing 1 with a slight variation of figure 1. As displayed, the bottom surface 10b of the wound dressing 1 is formed by a facing layer 5 with the application aid 6 attached to this facing layer 5. In contrast to figure 1, the application aid 6 in this embodiment forms two discontinuous stripes of pressure sensitive glue. These discontinuous stripes of pressure sensitive glue are covered by a protective film not shown in figure 2 which has to be removed before using the wound dressing 1. The stripes again extend along the long sides of the opposite edges 11 of the bottom surface 10b of the wound dressing 1. On the one hand, the surface of the pressure sensitive glue should suffice to guarantee a sufficient adhesion of the wound dressing 1 onto the patient's skin. On the other hand, the reduced surface of the pressure sensitive glue due to the discontinuous stripes should provide both a reduced pain in case of the removal of the wound dressing 1 and an improved transport of exudate from the wound to the absorbent core.

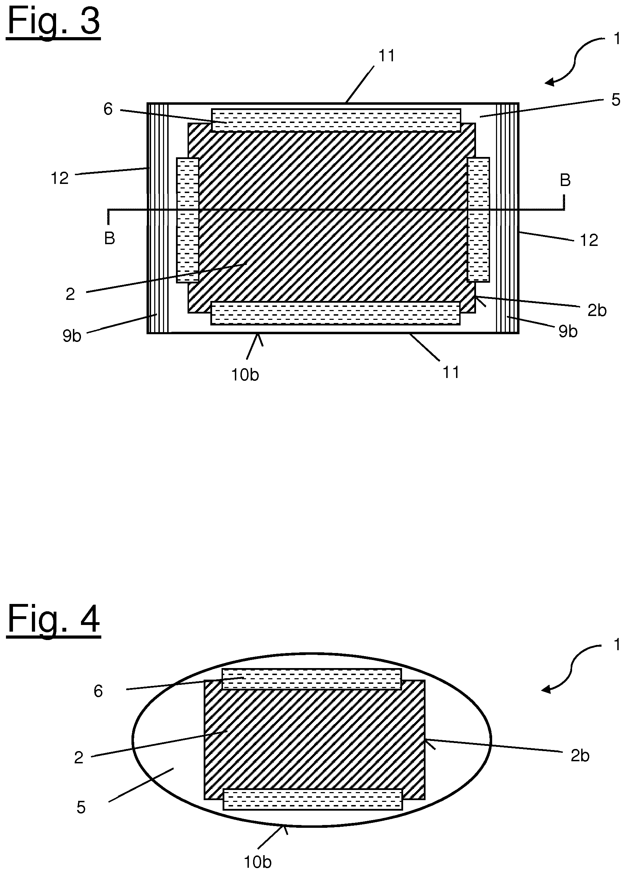

In figure 3, a view onto the bottom surface 10b of another embodiment of a wound dressing 1 is shown. Herein, the application aid 6 forms four stripes of pressure sensitive glue, wherein two stripes are arranged parallel to each other, respectively. Each pair of stripes being parallel to each other extend along the long or short sides of the opposite edges 11, 12 of the bottom surface 10b of the wound dressing 1. The stripes of pressure sensitive glue may form a complete frame overlapping with all the edges of the bottom surface 2b of the absorbent core 2. But they may also be discontinuous such that they only partly overlap with the edges of the bottom surface 2b of the absorbent core 2, like it is displayed in figure 3. The bottom surface 2b of the absorbent core 2 being in contact with the facing layer 5 is also shown in figure 3 although lying in a deeper plane with respect to the facing layer 5. It is displayed in order to illustrate the area ratio between the bottom surface 2b of the absorbent core 2 and the application aid 6 and, in particular, the covering of the bottom surface 2b of the absorbent core 2 by the application aid 6.

In the embodiment of Figure 3 the four stripes of pressure sensitive glue are arranged such that less than 20% of the surface, on which the pressure sensitive glue extends, overlaps with, i.e. covers, the bottom surface 2b of the absorbent core 2. This requirement provides that the exudate transport from the wound to the absorbent core 2 is blocked as little as possible by the pressure sensitive glue.

Figure 4 shows an alternative to a rectangular shape of the surface of a wound dressing 1. In this embodiment, the bottom surface 10b of the wound dressing 1 forms an ellipse while the bottom surface 2b of the absorbent core 2 still has a rectangular form. Such an elliptic shape of the wound dressing 1 may reduce side leakage from the exudate, which has already been absorbed by the absorbent core 2 of the wound dressing 1. Due to the elliptical shape the facing layer 5 of the wound dressing 1 provides a laterally larger extension compared to a rectangular shape of the surface of a wound dressing such that the retention of exudate can be enhanced. The application aid 6 in this embodiment consists of two parallel stripes of pressure sensitive glue which extend parallel to the long sides of the opposite edges of the bottom surface 2b of the absorbent core 2. These stripes can either be continuous or discontinuous.

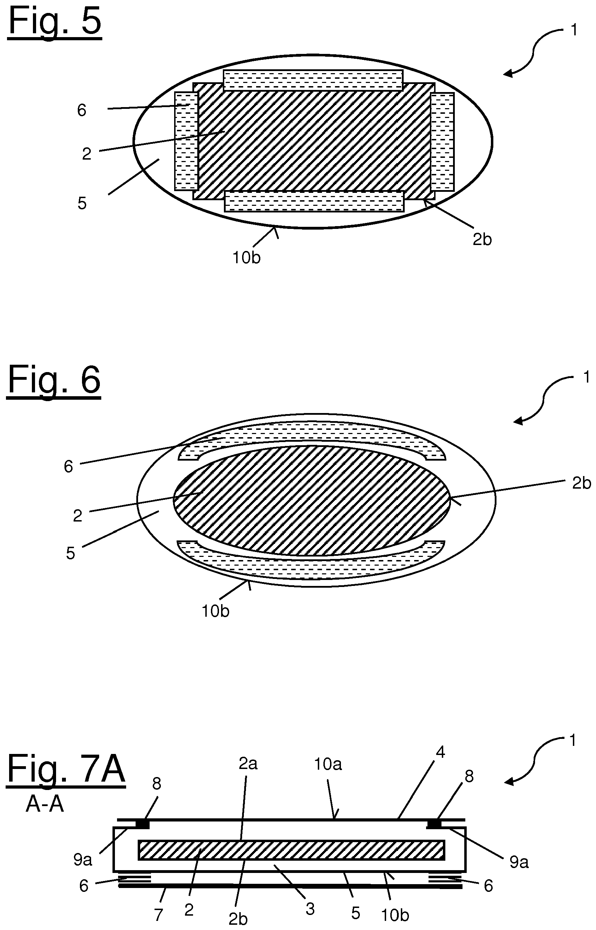

In contrast to figure 4, another embodiment of the bottom surface of a wound dressing 1, as it is shown in figure 5, provides an application aid 6 consisting of four stripes of pressure sensitive glue. These stripes extend parallel to the long sides and parallel to the short sides of the opposite edges of the bottom surface 2b of the absorbent core 2, wherein two of them are parallel to each other, respectively. The four stripes may form a frame, thereby covering all the edges of the bottom surface 2b of the absorbent core 2. However, they may also form discontinuous stripes such that there is no complete overlap with the edges of the bottom surface 2b of the absorbent core 2. The stripes of pressure sensitive glue are arranged such on the bottom surface 2b of the absorbent core 2 that they cover an area of the bottom surface of the absorbent core which is smaller than 10%.This arrangement results in an enhancement of transport of exudate from the wound to the absorbent core since an increased area absorbing the exudate is provided.

Yet another embodiment of the bottom surface 10b of the wound dressing 1 is shown in figure 6. Both the bottom surface 10b of the wound dressing 1 and the bottom surface 2b of the absorbent core 2 are elliptically shaped. The application aid 6 forms two curved stripes of pressure sensitive glue adapting to the shape of the elliptical form of the bottom surface 2b of the absorbent core 2.

In one embodiment, these curved stripes of pressure sensitive glue overlap at least partly with the edges of the bottom surface 2b of the absorbent core 2. In another embodiment, there is no overlap between the curved stripes of the pressure sensitive glue and the edges of the bottom surface 2b of the absorbent core 2. Thus, the transport of exudate from the wound to the absorbent core 2 is not limited at all by such a design of an application aid 6.

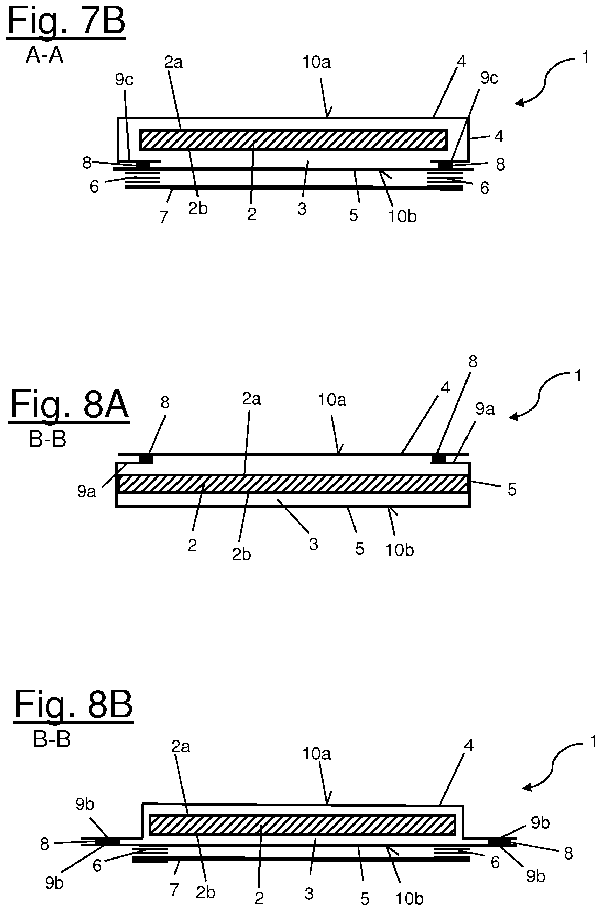

The wound dressings 1 of figures 1 to 6 consist of a layered structure each, which are best understood, when considered with reference to the cross-sectional drawings of figures 7A, 7B as well as 8A, 8B. These figures represent alternative embodiments of the layered structures comprised by the wound dressings 1.

Figure 7A shows a schematic cross sectional view long line AA of figure 1. The wound dressing 1 of figure 7A comprises an absorbent core 2 which is enclosed by a cover 3 having a backing layer 4 and a facing layer 5. Both the backing layer 4 and the facing layer 5 can either have a single-layer or a multilayer structure.

The facing layer 5 in this particular embodiment is made of a hydrophilic non-woven fabric consisting of polypropylene fibres in order to provide a good transport of exudate from a wound to the absorbent core 2.

As an example, the backing layer 4 serves as a clothing protection and is made of a breathable hydrophobic non-woven fabric based on polypropylene. The non-woven has a hydrohead of 50 cm/H₂O. This backing layer 4 allows breathing of the wound while simultaneously preventing wound exudates from exiting the wound dressing 1 and contaminating a patient's clothing.

According to figure 7A, the backing layer 4 and the facing layer 5 have been joint together by two seams 8 extending along the top surface 2a of the absorbent core 2. In order to do so, the facing layer 5 has been folded in parallel to its long edges such that it also extends above the absorbent core 2 and it partly covers the top surface 2a of the absorbent core 2. Furthermore, the facing layer 5 entirely covers the bottom surface 2b of the absorbent core 2.

A location of the seams 8 in an area above the absorbent core 2, i.e. in a position where the backing layer 4 and the facing layer 5 overlap above the absorbent core 2, has the advantage that no reddening due to stiff edges along the long side of the wound dressing 1 occurs. When considered in a cross-section along line A-A of figure 1, the wound dressing 1 thus has a tubular structure, as shown in figure 7A.

Different designs of how to locate the application aid 6 on the facing layer 5, which covers the bottom surface 2b of the absorbent core 2 are depicted in figures 1 to 6. Some of these designs constitute an application aid 6 in form of two stripes of pressure sensitive glue being parallel to each other and extending along the long sides of opposite edges 11 of the bottom surface of the facing layer 5. In other embodiments, the application aid 6 comprises four stripes of pressure sensitive glue, wherein two stripes are parallel to each other, respectively. One pair of these parallel stripes extends on the long sides and the other pair of these parallel stripes extends on the short sides of opposite edges 11, 12 of the bottom surface of the facing layer 5. The respective stripes may either extend continuously or discontinuously. However, less than 20%, of the surface, on which the respective stripes extend, overlaps with the bottom surface 2b of the absorbent core 2, i.e. the covering of the bottom surface 2b of the absorbent core 2 by the application aid 6 should be as small as possible in order to prevent a blocking of the transport of exudate from the wound to the absorbent core 2.

These different approaches where to locate the application aid may be combined with different approaches how to provide the layered structure containing the facing layer, the absorbent core and the backing layer in order to form the wound dressing as depicted in the cross sectional drawings of figures 7A, 7B as well as 8A, 8B and 10.

In figure 7B, an alternative embodiment of the wound dressing 1 with an alternative arrangement of the layered structure is displayed. The cross-sectional view along a line corresponding to line A-A of figure 1 again illustrates a tubular structure. However, in this alternative embodiment, the backing layer 4 has been folded in parallel to its long edges such that it extends beneath the absorbent core 2 and partly covers the bottom surface 2b of the absorbent core 2. Furthermore, the backing layer 4 entirely covers the top surface 2a of the absorbent core 2. The facing layer 5 is attached to the folded sections of the backing layer 4 by a seam 8. When the wound dressing 1 is in use, the facing layer 5 and partly also the backing layer 4 with its folded sections therefore face the wound.

In comparison to figure 7A, the application aid 6 in addition to its location on the facing layer 5 may now partly be located on top of the folded sections of the backing layer 4 covering the bottom surface 2b of the absorbent core 2. Different designs of how to arrange the application aid 6 onto the facing layer 5 and maybe partly onto the folded sections of the backing layer 4 can be realized. Some of these possibilities are shown in the schematic views onto the bottom surface 10b of the wound dressing 1 according to figures 1 to 6 and are described in the corresponding description of the figures. All of these designs, but also designs going beyond, can be combined with the alternative cross-sectional view of the wound dressing 1 shown in figure 7B. Since the plane of the bottom surface 10b of the wound dressing 1 and the plane of the cross-sectional view of the wound dressing 1 are perpendicular to each other, they can be combined with each other in various configurations.

Figure 8A shows a cross-sectional view of a wound dressing 1 along line B-B of figure 1 being perpendicular to the line A-A as indicated in figure 1. The wound dressing 1 of figure 8A comprises an absorbent core 2 which is enclosed by a cover 3 having a backing layer 4 and a facing layer 5. Both the backing layer 4 and the facing layer 5 can either have a single-layer or a multilayer structure.

The facing layer 5 in this particular embodiment is made of a hydrophilic non-woven fabric consisting of polypropylene fibres in order to provide a good transport of exudate from a wound to the absorbent core 2.

As an example, the backing layer 4 serves as a clothing protection and is made of a breathable hydrophobic non-woven fabric based on polypropylene. The non-woven has a hydrohead of 50 cm/H2O. This backing layer 4 allows breathing of the wound while simultaneously preventing wound exudates from exiting the wound dressing 1 and contaminating a patient's clothing.

In figure 8A, the backing layer 4 and the facing layer 5 have been joint together by together by two folded and sealed sections 9a on the short edges of the wound dressing which extend along the top surface 2a of the absorbent core 2. In order to do so, the facing layer 5 has been folded in parallel to its short edges such that it also extends above the absorbent core 2 and it partly covers the top surface 2a of the absorbent core 2. Furthermore, the facing layer 5 entirely covers the bottom surface 2b of the absorbent core 2. The backing layer 4 covers the top surface 2a of the absorbent core 2 and is sealed with the folded sections of the facing layer 5.

A location of the folded and sealed sections 9a in an area above the absorbent core 2, i.e. in a position where the backing layer 4 and the facing layer 5 overlap above the absorbent core 2, has the advantage that no reddening due to stiff edges along the short side of the wound dressing 1 occurs. When considered in a cross-section along line B-B of figure 1, the wound dressing 1 thus has a tubular structure, as shown in figure 8A.

Assuming that the wound dressing 1 of figure 1 comprises cross-sections along lines A-A and B-B of figures 7A and 8A none of the seams faces towards the skin.

Figure 8B shows a cross-sectional view of an alternative embodiment of a wound dressing 1 along line B-B of figure 3 being perpendicular to the line A-A as indicated in figure 1. In comparison to figure 8A, the backing layer 4 and the facing layer 5 have been joint together by two cross-seams 8 on the short edges 11 of the wound dressing 1. These cross-seams 8 extend along both lateral surfaces, which are parallel to each other and perpendicular to the plane of the top surface 2a and the bottom surface 2b of the absorbent core 2 and outside the absorbent core 2, thereby forming a sandwich design. The seam 8, when viewed in a bottom view is formed on a section 9b of the facing layer extending beyond the edges of the absorbent core 2 such that it does not come into direct contact with the patient's wound, i.e. there is a distance between each cross-seam 8 and the wound.

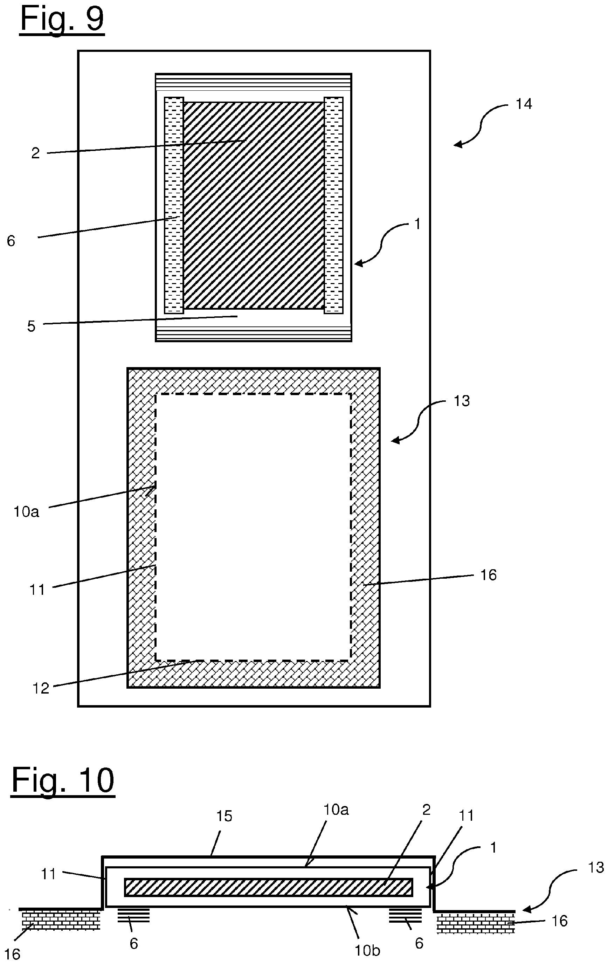

Figure 9 displays a schematic view onto the bottom surface of a wound dressing kit 14 comprising a wound dressing 1 according to figure 1 of the present invention and, furthermore, an additional permanent fixation 13. After the application of the wound dressing 1 onto the wound the permanent fixation 13 is placed on top of the wound dressing 1 such that it covers the top surface 10a of the wound dressing 1 completely extending at least 20 mm beyond the edges 11, 12 of the top surface 10a of the wound dressing 1, thereby forming a border around the wound dressing 1. In order to illustrate the area ratio of the top/bottom surface of the permanent fixation 13 compared to the top/bottom surface of the wound dressing 1, the permanent fixation 13 in figure 8 is transparently depicted such that the area of the permanent fixation 13 occupied by the top/bottom surface of the wound dressing in case of application as well as the border becomes visible. This border attaches the permanent fixation 13 onto the skin by a pressure sensitive glue which covers the bottom surface of the permanent fixation 13 facing to the patient's skin. The pressure sensitive glue used for the permanent fixation 13 hereby has a stronger adhesion to the skin than the pressure sensitive glue used for the application aid 6.

In this embodiment, the pressure sensitive glue covering the bottom surface of the permanent fixation 13 is a nolax M11.228 glue, a pressure sensitive hot melt adhesive non-irritating to the skin which bases on thermally rearranged (TR) polymers. According to the corresponding technical data sheet, which is published on the nolax webpage http://nolax.com/technische-datenblaetter/e/e_nolax_m11.228.pdf, the nolax M11.228 has a 180° peel adhesion of 1.04 N/mm. Thus, it exceeds by far the respective value obtained when measuring the 180° peel adhesion of the pressure sensitive glue, which is preferably used for the application aid 6, as the example of the nolax M11.1289 glue illustrates. Due to the strong adhesion of the nolax M11.228 it is well suited for a permanent fixation on the skin. According to the FTM 9, providing a means of measuring the tack of an adhesive, the loop tack value results in a maximum force of 38 N which is necessary in order to separate a loop of the adhesive tape material from a polished glass plate, to which the loop has been brought in contact with before. This high tack value of the pressure sensitive glue, exceeding by far the tack value of a pressure sensitive glue used for the application aid 6 guarantees a reliable permanent fixation on the skin and prevents a later slipping of the wound dressing 1 on the skin. Furthermore, the rolling ball tack test, used for quantifying the ability of an adhesive to adhere quickly to another surface, yields a distance of larger than 80 cm covered by a stainless steel ball rolling along the nolax M11.228 adhesive until the ball is stopped due to its adhesion to this specific nolax pressure sensitive hot melt.

However, the nolax M11.228 only represents one explicit example of a pressure sensitive glue used for the permanent fixation 13 in terms of the present invention. Other types of pressure sensitive glues may also be used for the permanent fixation 13 as long as they meet the necessary requirements, e.g. a high adhesion, according to the present invention.

In this embodiment, the covering layer of the permanent fixation 13 is made of a transparent polyurethane film coated with the above-mentioned nolax M11.228 pressure sensitive hot melt adhesive. The film is impermeable to germs as well as impermeable to moisture allowing the patients to wash or to take a shower. The stretchable film having a high elasticity conforms smoothly to body contours and adapts well to movements.

Figure 10 shows a cross-sectional view of the wound dressing 1 in combination with the permanent fixation 13 of figure 8. It represents a simplified cross-sectional view compared to figures 7A and 7B limited to the most relevant features. The covering layer 15 of the permanent fixation 13 covers entirely the top surface 10a of the wound dressing 1 and extends at least 20 mm beyond the long sides of opposite edges 11 of the top surface 10a of a wound dressing 1 as well as beyond the short sides of opposite edges 12 of the top surface 10a of a wound dressing 1, not shown in figure 9 since they are only presentable in a plane perpendicular to the demonstrated one. The bottom surface of the covering layer 15 facing to the patient's skin in case of application is covered by a pressure sensitive glue. The sections of the covering layer 15 which extend beyond the top surface 10a of the wound dressing 1, are attached to the patient's skin via the pressure sensitive glue.

In this embodiment, the covering layer 15 of the permanent fixation 13 is made of a transparent polyethylene film coated with a polyacrylate adhesive non-irritating to the skin. The thin film is impermeable to germs as well as impermeable to moisture allowing the patients to wash or to take a shower. The stretchable film having a high elasticity conforms smoothly to body contours and adapts well to movements. The polyacrylate adhesive serves as a link between the covering layer 15 and the patient's skin. It has a high adhesion to the patient's skin, thus, it provides a permanent fixation on the latter according to the present invention.

For purposes of original disclosure, it is pointed out that all features which are apparent for a person skilled in the art from the present description, the figures and the claims, even if they have only been described with further features, could be combined on their own or together with all the combinations of the features disclosed herein, if not excluded explicitly or technically impossible. A comprehensive explicit description of all possible combinations of features is only omitted in order to provide readability of the description.

### Reference numbers:

- 1: wound dressing
- 2: absorbent core
- 2a, 2b: top, bottom surface of absorbent core
- 3: cover
- 4: backing layer
- 5: facing layer
- 6: pressure sensitive glue
- 7: protective film
- 8: glue
- 9a: folded section of the facing layer
- 9b: non-folded glued section of the facing layer
- 9c: folded section of the backing layer
- 10a, 10b: top, bottom surface of wound dressing
- 11, 12: long, short sides of opposite edges of the top/bottom surface of a wound dressing
- 13: permanent fixation
- 14: wound dressing kit
- 15: covering layer
- 16: highly adhesive glue

## Claims

1. A wound dressing (1) having a top surface (10a) and a bottom surface (10b) with an absorbent core (2) having a top surface (2a) and a bottom surface (2b) and comprising a super absorbent substance and a cover (3),
wherein the absorbent core (2) is enclosed by the cover (3),
**characterized in that**
the wound dressing (1) has an application aid (6) in form of a pressure sensitive glue, wherein the pressure sensitive glue extends on an outer surface of the cover (3) facing towards a wound, when the wound dressing (1) is in use,
wherein the total surface covered by the pressure sensitive glue is smaller than the bottom surface (2b) of the absorbent core (2),
wherein a 180° peel adhesion of the pressure sensitive glue is smaller than or equal to 0.68 N/mm and exceeds 0.004 N/mm.

2. A wound dressing (1) according to the previous claim, **characterized in that** the 180° peel adhesion of the pressure sensitive glue is smaller than or equal to 0.2 N/mm.

3. A wound dressing (1) according to one of the previous claims, **characterized in that** a rolling ball on the pressure sensitive glue according to the PSTC 6 test will be stopped due to adhesion after 8 cm or less such that the pressure sensitive glue adheres quickly to the skin when applied to a wound.

4. A wound dressing (1) according to one of the previous claims, **characterized in that** a tack of the pressure sensitive tape determined in the framework of the FINAT 9 test, wherein the test is carried out with a contact speed as well as a separation speed of 5 mm/sec, and a width of the pressure sensitive glue of 25 mm as well as a length of the pressure sensitive glue of 150 mm, is smaller than or equal to 25 N.

5. A wound dressing (1) according to one of the previous claims, **characterized in that** the pressure sensitive glue is a hot melt.

6. A wound dressing (1) according to one of the previous claims, **characterized in that** the pressure sensitive glue is a hot melt comprising at least one of a group consisting of a thermoplastic elastomer, an acrylic, an ethylene-acrylate-elastomer, a polyolefin (PO), a low density polyethylene (LDPE) or a high density polyethylene (HDPE), a polybutene-1, a polyamide (PA), a polyester-elastomer (TPE-E), a polyurethane-elastomer (TPE-U), a styrene-butadiene-styrene (SBS), a styrene-isoprene-styrene (SIS), a styrene-ethylene/butylene-styrene (SEBS) or a styrene-ethylene/propylene (SEP) or a combination thereof.

7. A wound dressing (1) according to one of the previous claims, **characterized in that** the surface of the pressure sensitive glue facing the skin during use of the wound dressing (1) and before application onto the skin is shielded by a protective film (7.

8. A wound dressing (1) according to one of the previous claims, **characterized in that** the pressure sensitive glue is arranged in the form of at least two stripes extending along the long sides or the short sides of opposite edges (11 or 12) of the bottom surface (10b) of the wound dressing (1).

9. A wound dressing (1) according to one of the previous claims, **characterized in that** the total surface of the outer surface of the cover (3) covered by the pressure sensitive glue is smaller than 30 per cent of the bottom surface (2b) of the absorbent core (2).

10. A wound dressing (1) according to one of the previous claims, **characterized in that** less than 40 per cent of the outer surface of the cover (3) covered by the pressure sensitive glue overlaps with the bottom surface (2b) of the absorbent core (2).

11. A wound dressing (1) according to one of the previous claims, **characterized in that** the pressure sensitive glue is arranged in form of two adhesive stripes extending parallel to each other along opposite edges of the bottom surface of the wound dressing (1).

12. A wound dressing (1) according to one of the previous claims, **characterized in that** the cover (3) comprises a facing layer (5) in form of either a single-layer or a multilayer and a backing layer (4) in form of either a single-layer or a multilayer, wherein the facing layer (5) faces the wound when the wound dressing (1) is in use, wherein the facing layer (5) covers the bottom surface (2b) of the absorbent core (2), wherein the facing layer (5) is folded towards the top surface (2a) of the absorbent core (2) forming two folded sections, wherein the backing layer (4) is attached to the folded sections of the facing layer (5) by a seam and wherein the application aid (6) is located on the facing layer (5).

13. A wound dressing (1) according to one of the previous claims, **characterized in that** the cover (3) comprises a facing layer (5) in form of either a single-layer or a multilayer and a backing layer (4) in form of either a single-layer or a multilayer, wherein the backing layer (4) covers the top surface (2a) of the absorbent core (2), wherein the backing layer (4) is folded towards the bottom surface (2b) of the absorbent core (2) forming two folded sections, wherein the facing layer (5) is attached to the folded sections of the backing layer (4) by a seam, wherein the facing layer (5) faces the wound when the wound dressing (1) is in use and wherein the application aid (6) is located on the facing layer (5) and/or on the folded sections of the backing layer (4) covering the bottom surface (2b) of the absorbent core (2).

14. A kit (14) comprising a wound dressing (1) according to one of the previous claims and a permanent fixation (13), **characterized in that** the permanent fixation (13) comprises a covering layer (15) and a pressure sensitive glue, wherein the permanent fixation (13) has a larger dimension than the top surface (10a) of the wound dressing (1) and wherein the pressure sensitive glue providing an adhesive contact between the covering layer (15) and the skin has a stronger adhesion to the skin than the pressure sensitive glue of the application aid (6).

## Patentansprüche

1. Wundauflage (1) mit einer oberen Fläche (1 Oa) und einer unteren Fläche (10b), welche einen absorbierenden Kern (2) mit einer oberen Fläche (2a) und einer unteren Fläche (2b) aufweist, und einer superabsorbierenden Substanz und einer Hülle (3),
wobei der absorbierende Kern (2) von der Hülle (3) umschlossen ist,
**dadurch gekennzeichnet, dass**
die Wundauflage (1) eine Applizierungshilfe (6) in Form eines selbstklebenden Klebstoffs aufweist,
wobei der selbstklebende Klebstoff sich auf einer einer Wunde zugewandten Außenfläche der Hülle (3) erstreckt, wenn die Wundauflage (1) verwendet wird,
wobei die von dem selbstklebenden Klebstoff bedeckte Gesamtfläche kleiner ist als die untere Fläche (2b) des absorbierenden Kerns (2),
wobei eine 180°-Schälkraft des selbstklebenden Klebstoffs kleiner oder gleich 0,68 N/mm und größer als 0,004 N/mm ist.

2. Wundauflage (1) nach dem vorangehenden Anspruch, **dadurch gekennzeichnet, dass** die 180°-Schälkraft des selbstklebenden Klebstoffs kleiner oder gleich 0,2 N/mm ist.

3. Wundauflage (1) nach einem der vorangehenden Ansprüche, **dadurch gekennzeichnet, dass** eine rollende Kugel auf dem selbstklebenden Klebstoff gemäß des PSTC 6 Tests aufgrund der Haftung nach 8 cm oder weniger gestoppt werden wird, so dass der selbstklebende Klebstoff schnell an der Haut haftet, wenn er auf eine Wunde aufgetragen wird.

4. Wundauflage (1) nach einem der vorangehenden Ansprüche, **dadurch gekennzeichnet, dass** eine Klebrigkeit des selbstklebenden Klebstoffs, welche im Rahmen des FINAT 9 Tests bestimmt wurde, wobei der Test mit einer Kontaktgeschwindigkeit sowie einer Trennungsgeschwindigkeit von 5 mm/sec ausgeführt wird, und einer Breite des selbstklebenden Klebstoffs von 25 mm sowie einer Länge des selbstklebenden Klebstoffs von 150 mm, kleiner oder gleich 25 N ist.

5. Wundauflage (1) nach einem der vorangehenden Ansprüche, **dadurch gekennzeichnet, dass** der selbstklebende Klebstoff ein Schmelzkleber ist.

6. Wundauflage (1) nach einem der vorangehenden Ansprüche, **dadurch gekennzeichnet, dass** der selbstklebende Klebstoff ein Schmelzkleber ist, welcher mindestens eines aus der Gruppe bestehend aus einem thermoplastischen Elastomer, einem Acryl, einem Ethylen-Acrylat-Elastomer, einem Polyolefin (PO), einem Polyethylen mit geringer Dichte (LDPE) oder einem Polyethylen mit hoher Dichte (HDPE), einem Polybuten-1, einem Polyamid (PA), einem Polyester-Elastomer (TPE-E), einem Polyurethan-Elastomer (TPE-U), einem Styrol-Butadien-Styrol (SBS), einem Styrol-Isopren-Styrol (SIS), einem Styrol-Ethylen/Butylen-Styrol (SEBS) oder einem Styrol-Ethylen/Propylen (SEP) oder einer Kombination davon aufweist.

7. Wundauflage (1) nach einem der vorangehenden Ansprüche, **dadurch gekennzeichnet, dass** die während der Anwendung der Wundauflage (1) der Haut zugewandte Fläche des selbstklebenden Klebstoffs vor der Anwendung auf die Haut durch eine Schutzschicht (7) abgeschirmt wird.

8. Wundauflage (1) nach einem der vorangehenden Ansprüche, **dadurch gekennzeichnet, dass** der selbstklebende Klebstoff in Form von mindestens zwei Streifen, welche sich entlang der langen Seiten oder der kurzen Seiten von gegenüberliegenden Kanten (11 oder 12) der unteren Fläche (10b) der Wundauflage (1) erstrecken, angeordnet ist.

9. Wundauflage (1) nach einem der vorangehenden Ansprüche, **dadurch gekennzeichnet, dass** die Gesamtfläche der Außenfläche der Hülle (3), welche von dem selbstklebenden Klebstoff bedeckt ist, kleiner als 30 % der unteren Fläche (2b) des absorbierenden Kerns (2) ist.

10. Wundauflage (1) nach einem der vorangehenden Ansprüche, **dadurch gekennzeichnet, dass** weniger als 40 % der Außenfläche der Hülle (3), welche von dem selbstklebenden Klebstoff bedeckt ist, mit der unteren Fläche (2b) des absorbierenden Kerns (2) überlappt.

11. Wundauflage (1) nach einem der vorangehenden Ansprüche, **dadurch gekennzeichnet, dass** der selbstklebende Klebstoff in Form von zwei Klebestreifen, welche sich parallel zueinander entlang gegenüberliegenden Kanten der unteren Fläche der Wundauflage (1) erstrecken, angeordnet ist.

12. Wundauflage (1) nach einem der vorangehenden Ansprüche, **dadurch gekennzeichnet, dass** die Hülle (3) eine Deckschicht (5) in Form von entweder einer Einzelschicht oder einer Mehrfachschicht und eine Trägerschicht (4) in Form von entweder einer Einzelschicht oder einer Mehrfachschicht aufweist, wobei die Deckschicht (5) der Wunde gegenüberliegt, wenn die Wundauflage (1) verwendet wird, wobei die Deckschicht (5) die untere Fläche (2b) des absorbierenden Kerns (2) abdeckt, wobei die Deckschicht (5) in Richtung der oberen Fläche (2a) des absorbierenden Kerns (2) so gefaltet ist, dass zwei gefaltete Abschnitte gebildet werden, wobei die Trägerschicht (4) an den gefalteten Abschnitte der Deckschicht (5) durch eine Naht befestigt ist und wobei die Applizierungshilfe (6) auf der Deckschicht (5) angeordnet ist.

13. Wundauflage (1) nach einem der vorangehenden Ansprüche, **dadurch gekennzeichnet, dass** die Hülle (3) eine Deckschicht (5) in Form von entweder einer Einzelschicht oder einer Mehrfachschicht und eine Trägerschicht (4) in Form von entweder einer Einzelschicht oder einer Mehrfachschicht aufweist, wobei die Trägerschicht (4) die obere Fläche (2a) des absorbierenden Kerns (2) abdeckt, wobei die Trägerschicht (4) in Richtung der unteren Fläche (2b) des absorbierenden Kerns (2) so gefaltet ist, dass zwei gefaltete Abschnitte geformt werden, wobei die Deckschicht (5) an den gefalteten Abschnitten der Trägerschicht (4) durch eine Naht befestigt ist, wobei die Deckschicht (5) der Wunde gegenüberliegt, wenn die Wundauflage (1) verwendet wird, und wobei die Applizierungshilfe (6) auf der Deckschicht (5) und/oder den gefalteten Abschnitten der Trägerschicht (4), welche die untere Seite (2b) des absorbierenden Kerns (2) bedeckt, angeordnet ist.

14. Set (14), welches eine Wundauflage (1) nach einem der vorangehenden Ansprüche und eine permanente Befestigung (13) aufweist, **dadurch gekennzeichnet, dass** die permanente Befestigung (13) eine Deckschicht (15) und einen selbstklebenden Klebstoff aufweist, wobei die permanente Befestigung (13) eine größere Dimension hat als die obere Fläche (10a) der Wundauflage (1) und wobei der selbstklebende Klebstoff, welcher einen haftenden Kontakt zwischen der Deckschicht (15) und der Haut bereitstellt, eine stärkere Haftung an der Haut hat als der selbstklebende Klebstoff der Applizierungshilfe (6).

## Revendications

1. Pansement de plaie (1) ayant une surface supérieure (10a) et une surface inférieure (10b) avec un coeur absorbant (2) ayant une surface supérieure (2a) et une surface inférieure (2b) et comprenant une substance superabsorbante et un cache (3),
dans lequel le coeur absorbant (2) est entouré par le cache (3), **caractérisé en ce que**
le pansement de plaie (1) a une aide à l'application (6) sous la forme d'une colle sensible à la pression,
dans lequel la colle sensible à la pression s'étend sur une surface externe du cache (3) faisant face à une plaie, lorsque le pansement de plaie (1) est utilisé,
dans lequel la surface totale couverte par la colle sensible à la pression est plus petite que la surface inférieure (2b) du coeur absorbant (2),
dans lequel une adhésion par pelage à 180° de la colle sensible à la pression est inférieure ou égale à 0,68 N/mm et supérieure à 0,004 N/mm.

2. Pansement de plaie (1) selon la revendication précédente, **caractérisé en ce que** l'adhésion par pelage à 180° de la colle sensible à la pression est inférieure ou égale à 0,2 N/mm.

3. Pansement de plaie (1) selon l'une des revendications précédentes, **caractérisé en ce qu'**une bille roulante sur la colle sensible à la pression selon le test PSTC 6 sera arrêtée en raison de l'adhésion après 8 cm ou moins de telle sorte que la colle sensible à la pression adhère rapidement à la peau lorsqu'elle est appliquée sur une plaie.

4. Pansement de plaie (1) selon l'une des revendications précédentes, **caractérisé en ce qu'**un pouvoir adhésif du ruban sensible à la pression déterminé dans le cadre du test FINAT 9, dans lequel le test est réalisé avec une vitesse de contact ainsi qu'une vitesse de séparation de 5 mm/s, et une largeur de la colle sensible à la pression de 25 mm ainsi qu'une longueur de la colle sensible à la pression de 150 mm, est inférieure ou égale à 25 N.

5. Pansement de plaie (1) selon l'une des revendications précédentes, **caractérisé en ce que** la colle sensible à la pression est une colle thermofusible.

6. Pansement de plaie (1) selon l'une des revendications précédentes, **caractérisé en ce que** la colle sensible à la pression est une colle thermofusible comprenant au moins l'un d'un groupe constitué par un élastomère thermoplastique, un acrylique, un élastomère acrylate-éthylène , une polyoléfine (PO), un polyéthylène basse densité (LDPE) ou un polyéthylène haute densité (HDPE), un polybutène-1, un polyamide (PA), un élastomère de polyester (TPE-E), un élastomère de polyuréthane (TPE-U), un styrène-butadiène-styrène (SBS), un styrène-isoprène-styrène (SIS), un styrène-éthylène/butylène-styrène (SEBS) ou un styrène-éthylène/propylène (SEP) ou une de leurs combinaisons.

7. Pansement de plaie (1) selon l'une des revendications précédentes, **caractérisé en ce que** la surface de la colle sensible à la pression faisant face à la peau pendant une utilisation du pansement de plaie (1) et avant l'application sur la peau est protégée par un film protecteur (7).

8. Pansement de plaie (1) selon l'une des revendications précédentes, **caractérisé en ce que** la colle sensible à la pression est agencée sous la forme d'au moins deux bandes s'étendant le long des côtés longs ou des côtés courts des bords opposés (11 ou 12) de la surface inférieure (10b) du pansement de plaie (1).

9. Pansement de plaie (1) selon l'une des revendications précédentes, **caractérisé en ce que** la surface totale de la surface externe du cache (3) couverte par la colle sensible à la pression est inférieure à 30 pour cent de la surface inférieure (2b) du coeur absorbant (2).

10. Pansement de plaie (1) selon l'une des revendications précédentes, **caractérisé en ce que** moins de 40 pour cent de la surface externe du cache (3) couverte par la colle sensible à la pression se chevauchent avec la surface inférieure (2b) du coeur absorbant (2).

11. Pansement de plaie (1) selon l'une des revendications précédentes, **caractérisé en ce que** la colle sensible à la pression est agencée sous la forme de deux bandes adhésives s'étendant parallèlement l'une à l'autre le long de bords opposés de la surface inférieure du pansement de plaie (1).

12. Pansement de plaie (1) selon l'une des revendications précédentes, **caractérisé en ce que** le cache (3) comprend une couche de revêtement (5) sous la forme soit d'une couche unique soit d'une multicouche et une couche de support (4) sous la forme soit d'une couche unique soit d'une multicouche, dans lequel la couche de revêtement (5) fait face à la plaie lorsque le pansement de plaie (1) est utilisé, dans lequel la couche de revêtement (5) recouvre la surface inférieure (2b) du coeur absorbant (2), dans lequel la couche de revêtement (5) est pliée vers la surface supérieure (2a) du coeur absorbant (2) en formant deux sections pliées, dans lequel la couche de support (4) est fixée aux sections pliées de la couche de revêtement (5) par une jointure et dans lequel l'aide à l'application (6) est située sur la couche de revêtement (5).

13. Pansement de plaie (1) selon l'une des revendications précédentes, **caractérisé en ce que** le cache (3) comprend une couche de revêtement (5) sous la forme soit d'une couche unique soit d'une multicouche et une couche de support (4) sous la forme soit d'une couche unique soit d'une multicouche, dans lequel la couche de support (4) recouvre la surface supérieure (2a) du coeur absorbant (2), dans lequel la couche de support (4) est pliée vers la surface inférieure (2b) du coeur absorbant (2) en formant deux sections pliées, dans lequel la couche de revêtement (5) est fixée aux sections pliées de la couche de support (4) par une jointure, dans lequel la couche de revêtement (5) fait face à la plaie lorsque le pansement de plaie (1) est utilisé et dans lequel l'aide à l'application (6) est située sur la couche de revêtement (5) et/ou sur les sections pliées de la couche de support (4) recouvrant la surface inférieure (2b) du coeur absorbant (2).

14. Kit (14) comprenant un pansement de plaie (1) selon l'une des revendications précédentes et une fixation permanente (13), **caractérisé en ce que** la fixation permanente (13) comprend une couche de recouvrement (15) et une colle sensible à la pression, dans lequel la fixation permanente (13) a une dimension plus grande que la surface supérieure (10a) du pansement de plaie (1) et dans lequel la colle sensible à la pression fournissant un contact adhésif entre la couche de recouvrement (15) et la peau a une adhésion plus forte sur la peau que la colle sensible à la pression de l'aide à l'application (6).
